(19) Europäisches Patentamt
European Patent Office
Office européen des brevets

(11) Publication number: **0 285 123 A2**

# (12) EUROPEAN PATENT APPLICATION

(21) Application number: **88105163.5**

(51) Int. Cl.[4]: **C12N 15/00**, C12P 21/00

(22) Date of filing: **30.03.88**

A request for correction of the originally filed description pages 9 (line 12 and 15), 17 (line 24), 21 (column T) and claim 1 (line 8) has been filed pursuant to Rule 88 EPC. A decision on the request will be taken during the proceedings before the Examining Division (Guidelines for Examination in the EPO, A-V, 2.2).

(30) Priority: **03.04.87 US 34819**

(43) Date of publication of application: **05.10.88 Bulletin 88/40**

(84) Designated Contracting States: **AT BE CH DE ES FR GB GR IT LI LU NL SE**

(71) Applicant: **SUOMEN SOKERI OY**
**Kyllikinportti 2**
**SF-00240 Helsinki(FI)**

(72) Inventor: **Lehtovaara, Päivi**
**Tehtaank. 4A2**
**SF-00140 Helsinki(FI)**
Inventor: **Knowles, Jonathan**
**Ritokalliontie 15A**
**SF-00330 Helsinki(FI)**
Inventor: **Koivula, Anu**
**Koillisväylä 2 A 24**
**SF-00200 Helsinki(FI)**
Inventor: **Bamford, Jaana**
**Kartanont. 14A15**
**SF-00440 Järvenpää(FI)**
Inventor: **Reinikainen, Tapani**
**Klaarant.7A10**
**SF-00200 Helsinki(FI)**

(74) Representative: **Masch, Karl Gerhard et al**
**Patentanwälte Andrejewski, Honke & Partner**
**Theaterplatz 3 Postfach 10 02 54**
**D-4300 Essen 1(DE)**

## (54) A method for complete mutagenesis of nucleic acids.

(57) The invention relates to methods for introducing all types of base substitution mutations randomly to any nucleic acid. The methods comprise

(a) preparation of the target nucleic acid to be mutagenized in the form of a single stranded template;

(b) extension of an oligonucleotide hybridized to this template to generate a population of molecules, each copied from the template, and comprising molecules terminating at all possible nucleotide positions within a predetermined region;

(c) misincorporation of nucleotides opposite to each position of the template using polymerase and the variable 3′ ends generated in (b) as primers;

(d) completion of the molecules to forms that can be amplified by molecular cloning in a suitable host; and

(e) expression of the mutagenized DNA using a suitable host vector system allowing the screening for an altered gene or gene product.

EP 0 285 123 A2

# A method for complete mutagenesis of nucleic acids

This invention relates to methods for introducing in vitro all types of base substitution mutations randomly to any nucleic acid. Using this method it is possible to develop novel polypeptides or improved genetic expression systems for biotechnical applications. The method can also be used for identification of functionally important regions in proteins and gene regulatory regions. In one specific aspect, this invention relates to the generation of a population of DNA molecules, copied from a template, which comprise molecules terminating at all possible nucleotide positions within a predetermined target region, misincorporating nucleotides opposite to each position on the template and completing said molecules to forms which can be amplified by molecular cloning, expressing the mutagenized DNA, and screening for an altered gene or gene product.

Proteins and peptides play a central role in all biotechnical and pharmaceutical applications. Naturally, the properties of a particular protein should be chosen such that it functions under the conditions of the specific application as well as possible. In practice this is often achieved by screening a number of organisms for one producing a protein with the most appropriate properties. However, enzymes and other polypeptides have been selected during natural evolution for a specific molecular structure which allows them to function optimally in their respective host cells, and to interact with many other components of the cell. Therefore a protein isolated from a living organism is very unlikely to be perfectly suited to use in a given application.

The specific requirements of various technological applications thus create needs for altering some of the natural properties of the polypeptides used. Optimal performance of an enzyme in a biotechnical process may require alterations in properties such as pH-optimum, pH stability, thermostability, stability against oxidation or denaturation, substrate specificity, turnover number, spectrum of products, inhibition properties etc.

The primary structure of a polypeptide, as coded for by its respective gene, contains the determinants for its folding to a specific three-dimensional structure, and also for all of its properties. Therefore, by changing the base sequence of the respective gene, all properties of a protein can be in principle modified towards a desired optimum. Gradual accumulation of random base substitution mutations is an essential mechanism creating genetic diversity, which serves as material for selection under various conditions during the natural evolution of proteins. Any method able to mimic this process would provide a good basis for development of novel proteins for tehnological applications. The essential features of such methods include the random and efficient generation of base substitutions and the optimization of the ratio between the twelve different types of base substitutions.

A specific replacement of only one functionally critical amino acid by site specific oligonucleotide mutagenesis (see, Carter, P., Bedouelle H. and Winter, G., Improved oligonucleotide site-directed mutagenesis using M13 vectors 13 Nucleic Acids Res. 4431-4443 (1985); Smith M., In vitro mutagenesis, 19 Ann. Rev. Genet. 423-462 (1985); Leatherbarrow R.J. and Fersht, A.R., Protein engineering, 1 Protein Engineering 7-16 (1986)) has been demonstrated to be sufficient to bring about the desired modification of protein properties in, for example, the following cases: an altered specificity of trypsin, an altered pH dependence or enhanced resistance to chemical oxidation of subtilisin and an increased thermostability of lysozyme or dihydrofolate reductase (Leatherbarrow and Fersht 1986). However, the above enzymes all belong to the exceptionally rare examples where extensive information is available concerning the enzymology and three dimensional structure. Even in such cases, the choice of a suitable amino acid replacement still often involves much trial and error. This is due to the fact that precise prediction of which mutation is required to bring about a desired change in the properties of a protein is usually impossible, since our knowledge concerning the natural laws governing the structure and function of proteins is still very perfunctory.

Application of heteroduplex or cassette type of oligonucleotide mutagenesis to introduce several different mutations to complete genes consisting of hundreds or thousands of nucleotides is extremely difficult and expensive if not impossible in practice (Hutchison, C.A., Nordeen S.K., Vogt, K. and Edgell, M.H., A complete library of point substitution mutations in the glucocorticoid response element of mouse mammary tumor virus , 83 Proc. Natl. Acad. Sci. USA, 710-714 (1986)).

The prior art describes two different methods which enable making mutations to long DNA targets: chemical mutagenesis (Shortle D., and Nathans, D. Local mutagenesis: a method for generating viral mutants with base substitutions in preselected regions of the viral genome, 75 Proc. Natl. Acad. Sci. USA, 2170-2174 (1978); Hirose S., Takeuchi, K. and Suzuki, Y. In vitro characterization of the fibroin gene promoter by the use of single-base substitution mutants, 79 Proc. Natl. Acad. Sci. USA 7258-7262 (1982))

and enzymatic misincorporation of a noncomplementary nucleotide or thionucleotide (Zakour R.A. and Loeb L.A. Site-specific mutagenesis by error-directed DNA synthesis, 295 Nature 708-710 (1982); Shortle D., Grisafi, P., Benkovic, S.J. and Botstein, D., Gap misrepair mutagenesis: efficient site-directed induction of transition, transversion and frameshift mutations in vitro, 79 Proc. Natl. Acad. Sci. USA, 1588-1592 (1982)). Using the chemical mutagenesis method it is only possible to obtain a few types of base substitutions with each type of chemical modification, and the distribution of the mutations tends to be clustered in "hot spots". The chemical approach has been recently modified to give a wider range of mutations by making use of several different chemicals and by separate mutagenesis of both strands of DNA (Myers R.M., Lerman, L.S. and Maniatis, T., A general method for saturation mutagenesis of cloned DNA fragments, 229 Science 242-247 (1985). This method, while giving all possible point mutations, is laborious, time-consuming and involves use of mutagenic chemicals which are hazardous to health. In addition, it is not possible to optimize precisely the relative ratio, distribution and efficiency of all the different classes of mutants.

Using the gap misrepair method as described in Botstein, D. and Shortle, D., Strategies and applications of in vitro mutagenesis, 229 Science 1191-1201 (1985); Shortle, D., and Lin, B., Genetic analysis of Staphylococcus nuclease: identification of three intragenic "global" suppressors of nuclease-minus mutations, 110 Genetics, 539-555 (1985), a single nick is introduced into a covalently closed circular plasmid with DNaseI. The nicks are enlarged to short gaps where the 3'OH serves as the primer for misincorporation by polymerase. The disadvantage of the gap misrepair method is that the vector is also mutagenized, which decreases the efficiency of the method for the target region, and requires a time-consuming recloning step for the target DNA. Moreover, since both strands of the plasmid are mutagenized simultaneously, strand selection methods cannot be used to increase the efficiency of mutagenesis. It is not possible to localize mutations only to the region of DNA under study, such as the coding region or the promoter.

These facts reduce the efficiency of the gap misrepair method for generating the mutant library. Assuming a vector size of 3 kb, an insert size of 1 kb and a 100% efficient method, the maximal theoretical yield of mutants is only 12.5%. When a 1000-nucleotide region is mutagenized, the number of different possible point substitution mutants is 3000. Using the gap misrepair method, a complete library would be comprised of 24,000 clones (assuming 100% efficiency). Because the method for identifying mutant proteins can be laborious, it is of crucial importance that the mutagenesis method is highly efficient so that the library containing all mutants is as small as possible.

We have developed a complete mutagenesis method which makes it possible to saturate any cloned gene with random mutations.

The complete mutagenesis method described in this application allows rapid and efficient mutagenesis of defined regions of DNA. Since the mutagenized DNA strand can efficiently be selected for by known methods, the complete library made with this method would comprise only 3000 clones (assuming 100% efficiency). The number of transformants which must be screened in order to find a specific mutant naturally increases considerably if the mutations are not generated randomly. These parameters can be optimized to acceptable levels in the method described here. The present invention has clear advantage over the other methods, because it enables rapid and efficient mutagenesis of the desired target only, leaving other vector elements unmutagenized.

The present invention contemplates a mutagenesis method which allows rapid and efficient mutagenesis of defined regions of DNA. In addition, this method results in the rapid and efficient mutagenesis of the desired target only, leaving other vector elements unmutagenized.

Specifically, the method contemplates the introduction of random point mutations to a nucleic acid by preparing a target nucleic acid in the form of a single stranded template, extending an oligonucleotide hybridized to the template to generate a population of DNA molecules, which comprise molecules terminating at all possible nucleotide positions within a predetermined region, and misincorporating nucleotides opposite to each position on the template. The DNA molecules so generated are amplified by molecular cloning, and then screened for an altered gene or gene product.

The invention also contemplates a method of mutagenesis for preparing novel polypeptides, including those not found in nature, and a mutagenesis method for altering the structure and function of nucleic acid regions which function in regulating gene expression.

Figure 1 is a schematic representation of the method for generating a library of mutant genes. Mutagenesis of template T's is shown. A similar strategy is used to mutagenize template A's, C's or G's in separate series of reactions. The template T positions to be mutagenized are determined by the limited elongation step. The oligonucleotide primer is elongated with polymerase in conditions where the concentration of dATP soon becomes limiting, so that the elongation will stop at different points of sequence where an A would be required. After removal of free nucleotides, misincorporation to the 3' ends is carried

out using reverse transcriptase and only dCTP, dGTP and TTP. The molecules are finally elongated to completely double stranded forms by using polymerase and all the four nucleotides, and the gaps are sealed with ligase.

Figure 1b shows a typical result from the analysis of base-specifically ending elongated primers. The products from the limited elongation step have been electrophoresed in 6% sequencing gels. The sizes of the fragments are given as the number of nucleotides from the 3′ end of the primer. Panel A: The single stranded uracil containing M13mp19 template has been elongated with Klenow polymerase using the primer GAAGGGCGATCGGTGCG. The limiting nucleotide $\alpha$ -$^{32}$P-dCTP (diluted with the nonradioactive nucleotide to about 30 Ci/mmol) was used at three different concentrations, 0.075 µM, 0.20 µM and 0.325 µM (lanes 1-3, respectively). Aliquots of the samples were analyzed on sequencing gel. In lanes 4-6 the limiting nucleotide was $\alpha$ -$^{32}$P-dGTP (about 30 Ci/mmol). The marker lanes C and G show the corresponding dideoxy sequencing reactions, respectively. $^{35}$S-label was used in the marker lanes. Panel B: Template DNA, here containing an insert coding for $\alpha$-amylase from Bacillus stearothermophilus, has been copied from two different 20-mer oligonucleotide primers. The limiting nucleotide, $\alpha$ -$^{35}$S-dATP (diluted to about 300 Ci/mmol), was here used at four different concentrations, 0.03 µM, 0.075 µM, 0.20 µM and 0.32 µM (increasing concentrations in lanes 1-4 from one primer, and in lanes 5-8 from another primer, respectively).

Figure 2 shows the region coding for the $\alpha$-fragment of E.coli $\beta$-galactosidase, present in the DNA of the phage M13mp19. The sequence to which the specific oligonucleotide primer GAAGGGCGATCGGTGCG is hybridized is underlined. The region within parentheses shows the multiple cloning site. The wild type DNA sequence shown on top of the amino acid sequence has been mutagenized by introducing mostly single base substitutions. At least 176 different base substitutions obtained are shown. X is a mutation demonstrated by single track sequencing only.

Figure 3 shows the recombinant phages M13mp18$\alpha$ and M13mp19 $\alpha$ containing the B. stearothermophilus $\alpha$-amylase gene.

Figure 4 shows the amino acid sequence of Bacillus stearothermophilus $\alpha$-amylase, using one-letter notations for the amino acids. Some of the amino acid changes found by sequencing of the mutants are shown on top of the wild type sequence.

Figure 5 shows the altered pH-dependence of the $\alpha$-amylase activity of the mutant Tyr 265 → Ser, as compared to the wild type enzyme.

The present invention is a complete mutagenesis method which makes it possible to saturate any cloned gene with random mutations. This on the average leads to one amino acid change per mutant of the respective protein. Novel mutant enzymes having properties closer to that of the ideal are found by screening clones expressing the mutagenized gene. To permit even elaborate screening procedures, it is important that the wild type background is as low as possible, that the mutagenesis method is able to target the mutations in such a manner that they are distributed evenly within the region of interest and not elsewhere, and that the balance between all the possible 12 base substitutions can be optimized.

Table 1 shows which amino acid changes can be made to proteins by introducing all possible 574 single base substitutions to the 64 different codons. Silent mutations with no effect at protein level are shown in parentheses. Table 2 summarizes the data of amino acid replacement in Table 1. By using a suitable screening technique the complete mutagenesis method would have generated and identified the following single base mutations: the trypsin with altered substrate specificity (Gly226 → Ala, Craik, C.S., Largman, C., Fletcher, T., Roczniak, S., Barr, P.J., Fletterick, R. and Rutter, W.J., Redesigning trypsin: alteration of substrate specificity, 228 Science, 291-297 (1985)), the subtilisin mutant resistant to chemical oxidation (Met 222 → Leu, Estel, D.A., Graycar, T.P. and Wells, J.A., Engineering an enzyme by site-directed mutagenesis to be resistant to chemical oxidation, 260 J. Biol. Chem., 6518-6521 (1985)), the thermostable lysozyme mutant Lys 54 → Thr, Perry, L.J. and Wetzel, R., Unpaired Cysteine-54 interferes with the ability of an engineered disulfide to stabilize T4 lysozyme, 25 Biochemistry, 773-739 (1986)) and the tRNA synthetase mutant with a hundredfold improved affinity for the substrate ATP (Thr51 → Pro, Wilkinson, A.J., Fersht, A.R., Blow, D.M., Carter, P. and Winter, G., A large increase in enzyme-substrate affinity by protein engineering 307 Nature, 187-188 (1984)).

Table 1

| Amino acid replacements obtainedAla | |
|---|---|
| GCU → $(Ala)_3$ | Val Asp Ser Pro Thr Gly |
| GCC → $(Ala)_3$ | Val Asp Ser Pro Thr Gly |
| GCA → $(Ala)_3$ | Val Glu Ser Pro Thr Gly |

| | |
|---|---|
| GCG → (Ala)$_3$ | Val Glu Ser Pro Thr Gly |
| Arg | |
| CGU → (Arg)$_3$ | Leu Pro His Cys Ser Gly |
| CGC → (Arg)$_2$ | Leu Pro His Cys Ser Gly Arg |
| CGA → (Arg)$_4$ | Leu Pro Gln term Gly |
| CGG → (Arg)$_4$ | Leu Pro Gln Trp Gly |
| AGA → (Arg)$_2$ | term Gly Ser$_2$ Ile Thr Lys |
| AGG → (Arg)$_2$ | Trp Gly Met Thr Lys Ser$_2$ |
| Asn | |
| AAU → (Asn) | Asp Tyr His Ile Thr Ser Lys$_2$ |
| AAC → (Asn) | Asp Tyr His Ile Thr Ser Lys$_2$ |
| Asp | |
| GAU → (Asp) | Val Ala Gly Glu$_2$ Tyr His Asn |
| GAC → (Asp) | Val Ala Gly Glu Tyr His Asn Glu |
| Cys | |
| UGU → (Cys) | Arg Ser Gly term Trp Phe Ser Tyr |
| UGC → (Cys) | Arg Ser Gly Phe Ser Tyr term Trp |
| Gln | |
| GAA → (Gln) | Arg Leu Pro His$_2$ term Lys Glu |
| CAG → (Gln) | Arg Leu Pro His$_2$ term Lys Glu |
| Glu | |
| GAA → (Glu) | Val Ala Gly Asp$_2$ term Gln Lys |
| GAG → (Glu) | Val Ala Gly Asp$_2$ term Gln Lys |
| Gly | |
| GGU → (Gly)$_3$ | Val Ala Asp Cys Arg Ser |
| GGC → (Gly)$_2$ | Val Ala Asp Cys Arg Ser Gly |
| GGA → (Gly)$_3$ | Val Ala Glu term Arg$_2$ |
| GGG → (Gly)$_3$ | Val Ala Glu Trp Arg$_2$ |
| His | |
| CAU → (His) | Arg Leu Pro Gln$_2$ Asn Tyr Asp |
| CAC → (His) | Arg Leu Pro Gln Asn Tyr Asp Gln |
| Ile | |
| AUU → (Ile)$_2$ | Val Phe Leu Thr Asn Ser Met |
| AUC → (Ile)$_2$ | Val Phe Leu Thr Asn Ser Met |
| AUA → (Ile)$_2$ | Val Leu$_2$ Thr Lys Arg Met |
| Leu | |
| UUA → (Leu)$_2$ | Ile Val Ser term$_2$ Phe$_2$ |
| UUG → (Leu)$_2$ | Met Val Ser term Trp Phe$_2$ |
| CUU → (Leu)$_3$ | Pro His Arg Ile Phe Val |
| CUC → (Leu)$_2$ | Pro His Arg Ile Phe Val Leu |
| CUA → (Leu)$_3$ | Pro Gln Arg Ile Val Leu |
| CUG → (Leu)$_4$ | Pro Gln Arg Met Val |
| Lys | |
| AAA → (Lys) | Glu term Gln Ile Thr Arg Asn$_2$ |
| AAG → (Lys) | Glu term Gln Met Thr Arg Asn$_2$ |
| Met | |
| AUG → (Val Leu$_2$ Thr Lys Arg Ile$_3$ | |

Phe

| | |
|---|---|
| UUU → (Phe) | Leu$_3$ Ile Val Ser Thr Cys |
| UUC → (Phe) | Leu$_3$ Ile Val Ser Thr Cys |

Pro

| | |
|---|---|
| CCU → (Pro)$_3$ | Leu His Ser Thr Arg Ala |
| CCC → (Pro)$_2$ | Leu His Ser Thr Arg Ala Pro |
| CCA → (Pro)$_3$ | Leu Gln Ser Thr Arg Ala |
| CCG → (Pro)$_3$ | Leu Gln Ser Thr Arg Ala |

Ser

| | |
|---|---|
| UCU → (Ser)$_3$ | Pro Thr Ala Phe Tyr Cys |
| UCC → (Ser)$_2$ | Pro Thr Ala Phe Tyr Cys Ser |
| UCA → (Ser)$_3$ | Pro Thr Ala term$_2$ Leu |
| UCG → (Ser)$_3$ | Pro Thr Ala term Leu Trp |
| AGU → (Ser) | Gly Cys Arg$_3$ Ile Thr Asn |
| AGC → (Ser) | Gly Cys Arg$_2$ Ile Thr Asn Arg |

Thr

| | |
|---|---|
| ACU → (Thr)$_3$ | Ala Ser Pro Ile Asn Ser |
| ACC → (Thr)$_2$ | Ala Ser Pro Ile Asn Ser Thr |
| ACA → (Thr)$_3$ | Ala Ser Pro Ile Lys Arg |
| ACG → (Thr)$_3$ | Ala Ser Pro Met Lys Arg |

Trp

UGC → (Arg$_2$ Gly Leu Ser term$_2$ Cys$_2$

Tyr

| | |
|---|---|
| UAU → (Tyr) | His Asn Asp Cys Phe Ser term$_2$ |
| UAC → (Tyr) | His Asn Asp Cys Phe Ser term$_2$ |

Val

| | |
|---|---|
| GUU → (Val)$_3$ | Ala Asp Gly Ile Leu Phe |
| GUC → (Val)$_2$ | Ala Asp Gly Ile Leu Phe Val |
| GUA → (Val)$_3$ | Ala Glu Gly Leu$_2$ Ile |
| GUG → (Val)$_3$ | Ala Glu Gly Met$_2$ Leu |

term

| | |
|---|---|
| UAA → (term)$_2$ | Gln Lys Glu Leu Ser Tyr$_2$ |
| UAG → (term) | Gln Lys Glu Leu Ser Tyr$_2$ Trp |
| UGA → (term) | Arg$_2$ Gly Cys$_2$ Trp Leu Ser |

Table 2

To: -->

| * | ** | From: | Ala | Asn | Arg | Asp | Cys | Gln | Glu | Gly | His | Ile | Leu | Lys | Met | Phe | Pro | Ser | Thr | Trp | Tyr | Val | term |
|---|---|---|---|---|---|---|---|---|---|---|---|---|---|---|---|---|---|---|---|---|---|---|---|
| 4 | 36 | Ala | 12 | | | 2 | | | 2 | 4 | | | | | | | 4 | 4 | 4 | | | 4 | |
| 2 | 18 | Asn | | 2 | | 2 | | | | | 2 | 2 | | 4 | | | | 2 | 2 | | 2 | | |
| 6 | 54 | Arg | | | 18 | | 2 | 2 | | 6 | 2 | 1 | 4 | 2 | 1 | | 4 | 6 | 2 | 2 | | | 2 |
| 2 | 18 | Agp | 2 | 2 | | 2 | | | 4 | 2 | 2 | | | | | | | | | | 2 | 2 | |
| 2 | 18 | Cys | | | 2 | | 2 | | | 2 | | | | | | 2 | | 4 | | 2 | 2 | | 2 |
| 2 | 18 | Gln | | | 2 | | | 2 | 2 | | 4 | | 2 | 2 | | | 2 | | | | | | 2 |
| 2 | 18 | Glu | 2 | | | 4 | | 2 | 2 | 2 | | | | 2 | | | | | | | | 2 | 2 |
| 4 | 36 | Gly | 4 | | 6 | 2 | 2 | | 2 | 12 | | | | | | | | 2 | | 1 | | 4 | 1 |
| 2 | 18 | His | | 2 | 2 | 2 | | 4 | | | 2 | | 2 | | | | 2 | | | | 2 | | |
| 3 | 27 | Ile | | 2 | 1 | | | | | | | 6 | 4 | 1 | 3 | 2 | | 2 | 3 | | | 3 | |
| 6 | 54 | Leu | | | 4 | | | 2 | | | 2 | 4 | 18 | | 2 | 6 | 4 | 2 | | 1 | | 6 | 3 |
| 2 | 18 | Lys | | 4 | 2 | | | 2 | 2 | | | 1 | | 2 | 1 | | | | 2 | | | | 2 |
| 1 | 9 | Met | | | 1 | | | | | | | 3 | 2 | 1 | | | | | 1 | | | 1 | |
| 2 | 18 | Phe | | | | | 2 | | | | | 2 | 6 | | | 2 | | 2 | | | 2 | 2 | |
| 4 | 36 | Pro | 4 | | 4 | | | 2 | | | 2 | | 4 | | | | 12 | 4 | 4 | | | | |
| 6 | 54 | Ser | 4 | 2 | 6 | | 4 | | | 2 | | 2 | 2 | | | 2 | 4 | 14 | 6 | 1 | 2 | 3 | |
| 4 | 36 | Thr | 4 | 2 | 2 | | | | | | | 3 | | 2 | 1 | | 4 | 6 | 12 | | | | |
| 1 | 9 | Trp | | | 2 | | 2 | | | 1 | | | 1 | | | | | 1 | | | | | 2 |
| 2 | 18 | Tyr | | 2 | | 2 | 2 | | | | 2 | | | | | 2 | | 2 | | | 2 | | 4 |
| 4 | 36 | Val | 4 | | | 2 | | | 2 | 4 | | 3 | 5 | | 2 | 2 | | | | | | 12 | |
| 3 | 27 | term | | | 2 | | 2 | 2 | 2 | 1 | | | 3 | 2 | | | | 3 | | 2 | 4 | | 4 |
| 64 | 576 | | | | | | | | | | | | | | | | | | | | | | |

* number of alternative codons

** number of different point mutations

The method comprises the following procedures. Firstly, a genetic library of mutant genes is generated in vitro (as shown by Fig. 1). Each mutant contains one or two point mutations and the complete library contains all of the possible single base mutations of the gene. The mutant library is then expressed in a suitable host vector system and mutant genes coding for example for enzymes with improved properties are identified on the basis of enzyme activity measurements. These mutants with improved properties can then be then remutagenized to isolate even better mutants. This process mimics the evolution of proteins in nature, but allows man to determine the precise basis for selection and can be accomplished very rapidly.

The first step, limited elongation of a primer, which is carried out in four separate reactions, generates a

population of molecules each copied from the template and comprising molecules terminating at all possible nucleotide positions within a predetermined region. The second step, enzymatic misincorporation, generates point mutations into this molecular population. It is possible to make all the twelve possible point mutations or only a desired subset of them. Any base will be mutagenized to any of the three other bases, and the frequency of the different base substitutions can be optimized. The position and length of the target sequence to be mutagenized can be varied. The third step involves completion of the mutagenized molecules to forms that can be then amplified and isolated by molecular cloning.

Mutant proteins with the desired properties are then identified on the basis of enzyme activity produced by the expression of the mutagenized genes in a suitable host. Specific conditions are designed to screen the transformants for the particular trait in question. For example, if a mutant enzyme with an increased thermo stability is screened for, the clones are first made to express the mutant enzymes under optimal expression conditions, and then aliquots of the mutant enzymes produced are subjected to the testing temperatures and finally to activity determinations. When a desired clone has been found, the precise mutation can be identified by sequencing the mutant nucleic acid if such identification is required.

Example 1: Total Mutagenesis of the α-Fragment of β-galactosidase from E-coli

The α-fragment of β-galactosidase is mutagenized to demonstrate how the method works to generate a library of mutant genes. The protein product can be directly screened for light blue and white mutant phenotypes. The region coding for the α-fragment of E. coli β-galactosidase is set forth in Figure 2 and is included in the DNA of the single stranded phage vector M13mp19 as described in Yanisch-Perron, C., Vieira, J. and Messing, J., Improved M13 phage cloning vectors and host strains: nucleotide sequences of the M13mp18 and pUC19 vectors, 33 Gene, 103-119 (1985), Messing, J. New M13 vectors for cloning, 101 Methods Enzymol., 20-78 (1983).

A uracil-containing, single stranded template of M13mp19 phage is prepared essentially as described in Kunkel, T.A., Rapid and efficient site-specific mutagenesis without phenotypic selection, 82 Proc. Natl. Acad. Sci. USA 488-492 (1985). The ung-dut-strain of E. coli, BW313, is grown overnight at 37°C in 20 ml of 2 × TY medium. Then 5 ml of the cells are diluted to 200 ml with 2 × TY and grown for 1/2 h. The cells are infected with M13mp19 phage at the multiplicity of infection of 5, and grown in 200 ml of 2 × TY supplemented with 0.25 ug/ml uridine for 4-6 hours at 37°C. The supernatant is used to infect cells again as previously described to obtain a second cycle of phage growth. This uracil-containing M13mp19 phage grows in the dut-ung-mutant strain BW 313 (typically to a titer of $4 \times 10^{10}$ -$1 \times 10^{11}$ plaque forming units/ml) but does not grow in the dut+ ung+ strain (typical titer is $5 \times 10^{4}$ -$2 \times 10^{6}$, representing phages lacking uracil in the genome).

Single stranded uracil-containing M13mp19 DNA is isolated as follows. The cells grown in 200 ml as described are pelleted for 10-20 min by centrifugation at 5000 rpm in a Sorvall GSA rotor, and the supernatant is made 0.5M in NaCl and 8% in polyethylene glycol ("PEG"). After 1 hour at 0°C it is centrifuged at +4°C for 15 min at 10,000 rpm (GSA), and the phage pellet is dissolved in 30 ml TE. PEG is added to 2% and NaCl to 0.5 M, and after 1 hour at 0°C the phage is pelleted again by centrifugation for 10 minutes at 15,000 rpm, +4°C in a Sorvall rotor SS-34. The pellet is resuspended in 3 ml of 10 mM Tris-HCl pH 8.0, 50 mM NaCl, 1 mM EDTA and extracted twice with an equal volume of phenol, then with chloroform: isoamylalcohol (24:1) and finally three times with ether. The DNA is precipitated with ethanol and redissolved in TE.

The 17-mer oligonucleotide primer (2 ug) GAAGGGCGATCGGTGCG is 5′ end labeled to a low specific activity with polynucleotide kinase using γ-$^{32}$p-ATP and ATP as described in Maniatis, T., Fritsch, E.F. and Sambrook, J., Molecular Cloning, Cold Spring Harbor Laboratory (1982), and the primer is purified in a Bio-Gel P-6 gel filtration column (1 × 20 cm) in TE. The peak fractions of the eluate are combined, made 2 M in ammonium acetate and the DNA is precipitated with ethanol.

The uracil-containing template M13mp19-U (8.5 pmol) and the 17-mer primer (17 pmol) in 30 - 60 µl 15 mM Tris-HCl pH 8.0, 7.5 mM $MgCl_2$ is heated for 15 min at 80°C and slowly cooled to about 30°C. The annealed template/primer mixture is diluted with 10 mM Tris-HCl pH 8.0, 5 mM $MgCl_2$ to a concentration of 0.1 pmol template/primer per µl.

Limited Elongation

315 µl of water and 60 µl 100 mM Tris-HCl, pH 8.0, 50 mM $MgCl_2$ are added to 60 µl of template/primer (6 pmol), and the sample is distributed into 12 tubes in 36 µl (0.5 pmol) aliquots. To these tubes the components are added as follows (either thionucleotide or ordinary nucleotide triphosphates can be used):

| | Tube number | | | | | |
|---|---|---|---|---|---|---|
| | 1 | 2 | 3 | 4 | 5 | 6 |
| 0.33 mM CGT mix | 8 µl | 8 µl | 8 µl | | | |
| 0.33 mM AGT mix | | | | 8 µl | 8 µl | 8 µl |
| Limiting Nucleotide: | | | | | | |
| 0.75 µM dATP | 5 µl | | | | | |
| 2.0 µM dATP | | 5 µl | | | | |
| 3.25 µM dATP | | | 5 µl | | | |
| 0.75 µM dCTP | | | | 5 µl | | |
| 2.0 µM dCTP | | | | | 5 µl | |
| 3.25 µM dCTP | | | | | | 5 µl |

| | Tube number | | | | | |
|---|---|---|---|---|---|---|
| | 7 | 8 | 9 | 10 | 11 | 12 |
| 0.33 mM ACT mix | 8 µl | 8 µl | 8 µl | | | |
| 0.33 mM ACG mix | | | | 8 µl | 8 µl | 8 µl |
| Limiting Nucleotide: | | | | | | |
| 0.75 µM dGTP | 5 µl | | | | | |
| 2.0 µM dGTP | | 5 µl | | | | |
| 3.25 µM dGTP | | | 5 µl | | | |
| 0.75 µM TTP | | | | 5 µl | | |
| 2.0 µM TTP | | | | | 5 µl | |
| 3.25 µM TTP | | | | | | 5 µl |

where the 0.33 mM CGT mix contains dCTP, dGTP and TTP each at 0.33 mM concentration; 0.33 mM AGT mix dATP, dGTP and TTP each at 0.33 mM; 0.33 mM ACT mix dATP, dCTP and TTP each at 0.33 mM; and 0.33 mM ACG mix dATP, dCTP and dGTP each at 0.33 mM concentration.

Finally 1 µl of Klenow polymerase (5 U) is added to each of the tubes 1 - 12, and the samples are incubated for 5 min at room temperature. To stop the reactions 3 µl of 0.3 M EDTA is added to each of the tubes 1 - 12.

The limited elongation can be monitored by electrophoresis of aliquots of the samples on a sequencing gel (Sanger et al., 1977). The labeling of the samples for this purpose is done either by end-labeling the primer with $\gamma$-$^{32}$p-dATP as described above or by incorporated some $\alpha$-$^{32}$p-dNTP (any nucleotide) during the limited elongation. A typical result from a limited elongation is shown in Fig. 1b. The extent of limited elongation is regulated by the concentration of the limiting nucleotide. Thus the resulting populations of extended molecules can be adjusted to have the 3′ ends at variable distances from the primer. Under the conditions described here, the combined molecular populations cover at least 200 bp from the primer.

After the limited elongation step the contents of tubes 1 - 3 are combined and labeled as A-, likewise those of tubes of 4 - 6 (C-), 7 - 9 (G-) and 10 - 12 (T). 12 ug carrier glycogen is added to each sample, and the samples are extracted twice with phenol, twice with ether, made 250 µl in 2M ammonium acetate and

precipitated with 650 µl of 94% ethanol. The pellets are dissolved in 200 µl TE, 50 ul 10M ammonium acetate is added and the precipitation step repeated. After three successive precipitations to efficiently remove free nucleotides, the samples are washed with 70% ethanol, dried briefly and dissolved in 10 mM Tris-HCl pH 8.3, 0.1 mM EDTA.

Misincorporation

The population of molecules in tube A-consists of primers elongated to a variable degree and hybridized to the template so that each 3′ end is next to a position where the template contains a T. See Figure 1. When these elongated primers are now further elongated with a reverse transcriptase in the presence of dCTP, dGTP and TTP but in the absence of the correct nucleotide dATP, wrong nucleotides will be incorporated instead of the correct one. Since the correct nucleotide is always known, the efficiency of the method can be increased by omitting that nucleotide from the misincorporation reaction. Klenow polymerase and the corresponding thionucleotides can also be used. The misincorporation step using three wrong nucleotides is carried out for each of the four samples A-, C-, G-and T-. The concentrations of the "wrong" nucleotides are optimized to obtain good equilibrium between the various misincorporations.

4.6 µl of 5x reverse transcriptase buffer [250 mM Tris-HCl, pH 8.3 (at 37°C when diluted to 50 mM), 30 mM $MgCl_2$, 300 mM KCl], 0.25 µl 10 mM dCTP, 0.5 µl 10 mM dGTP, 0.25 µl 10 mM TTP, 0.25 µl 0.1 M dithiothreitol, 1.25 µl 1.75 mg/ml bovine serum albumin and 0.5 µl reverse transcriptase (12 - 25 U/µl) are added to 15 µl of DNA in tube A-, containing about 0.5 pmol of template/elongated primer. The sample is incubated at 37°C or at 42°C for 3 hours, and an additional 0.5 µl of reverse transcriptase is added after 1 1/2 h. After 3 hours of reaction 1.25 µl of a mixture containing all the four nucleotides at 10 mM concentration and 0.2 µl of reverse transcriptase are added and incubated at 42°C for 30 min. The sample is then cooled to 0°C. To elongate the newly synthesized strand to completion, the following components are added to each of the samples A-, C-, G-and T-: 1 µl 10 mM ATP, 5 µl of 5x reverse transcriptase buffer, 16 µl water, 2.5 µl T4 ligase (6 U/µl), 2 µl p32 (protein p32 from phage T4, 2.5 mg/ml) and 0.5 µl of T4 DNA-polymerase (10 U/µl). The samples are incubated at 14°C overnight or for a few hours. Alternatively, the final elongation step can be performed with 0,7 µl Klenow polymerase (5U/µl, Boehringer) in the conditions described above, but leaving out T4 DNA polymerase and p32.

Competent JM109 cells (see, Hanahan, D., Techniques for transformation of E. coli. In: DNA cloning, volume I (Glover, D.M., ed.), IRL Press, Oxford and Washington, pp. 109-136 (1985)) are transformed with the above samples using 0.3 ng, 3 ng and 50 ng of DNA per transformation. Control transformations are done with the single stranded uracil-containing template, and with both single stranded and double stranded wild type M13mp19 template. The cells are grown on plates containing IPTG and X-gal (as in Messing, 1983 (see above)). Typical transformation frequencies of the samples are around $10^5$ transformants/µg, the uracil-template alone gives 10 - 60 transformants/µg, the single stranded wild type M13mp19 about $10^5$/µg and the double stranded wild type M13mp19 typically $5 \times 10^6$ -$1 \times 10^7$/µg.

Some of the mutations made to the α-fragment of the E. coli β-galactosidase result in the formation of functionally defective β-galactosidase and thus lead to a white or light blue phenotype of plaques on IPTG and X-gal containing indicator plates. Blue plaques represent either wild type α-fragment sequences, silent mutations where a nucleotide change does not lead to an amino acid change, or mutations where the resulting amino acid does not affect the activity of the enzyme towards synthetic substrate X-gal. In order to characterize the precise distribution, frequence, nature and equilibrium of the mutations obtained, several phage DNAs from the white, light blue and blue plaques from various mutagenesis experiments are isolated and sequenced using the same primer as for the limited elongation step.

The mutations in the DNA isolated from the white and light blue plaques show which codon changes lead to functional changes in the enzyme. Phenotypically silent mutations from the blue plaque DNAs are distributed essentially in a random fashion. Fig. 2 shows the target α-fragment sequence and the various base substitutions found in the mutants. The primary target covered about 100 nucleotides from the primer. The region in parentheses is the polylinker region of M13mp19. In several experiments the phenotypic change was selected for, which explains the enrichment of certain point mutations. All the twelve possible types of point mutations have been obtained, namely:

$$A \rightarrow \begin{matrix}C\\G,\\T\end{matrix} \quad C \rightarrow \begin{matrix}A\\G,\\T\end{matrix} \quad G \rightarrow \begin{matrix}A\\C\\T\end{matrix} \text{ and } T \rightarrow \begin{matrix}A\\C.\\G\end{matrix}$$

Altogether 108 different nucleotides in the target DNA have been changed, and because more than one type of mutation has been obtained at many positions, the total number of different mutations obtained is at least 176. In an optimized system this result is achieved in one experiment, where the limited elongation, misincorporation and complete elongation steps can be performed in two days, and mutants obtained on the third day.

Table 3

<u>Mutagenesis of template residues T, A, C and G</u>

| Template residues mutagenized | Misincorporation conditions | Yield of mutants (from sequencing data) | Average No. of mutations per template | Ratio of different mutations |
|---|---|---|---|---|
| T<br>(A-reaction) | dCTP 100 µM<br>dGTP 100 µM<br>TTP 100 µM | 90 % | total: 3.0<br>blue: 2.4<br>white 3.6 | white<br>T -> G 1<br>C 33<br>A 1 |
| | dCTP 100 µM<br>dGTP 0.2 µM<br>TTP 100 | 42 % | total: 1.4<br>blue: 1.3<br>white: 2.3 | blue white<br>T -> G 5 4<br>C 10 20<br>A 3 8 |
| A<br>(T-reaction) | dATP 100 µM<br>dCTP 100 µM<br>dGTP 100 µM | 27 % | total: 1.1<br>blue: 1.1<br>white: 1.6 | blue white<br>A -> T 15 30<br>G 9 32<br>C 2 17<br>(26 deletions) |
| C<br>(G-reaction) | dATP 100 µM<br>dCTP 100 µM<br>TTP 100 µM | 59 % | total: 1.7<br>blue: 1.7<br>white 1.6 | blue white<br>C -> T 14 15<br>G 0 0<br>A 3 9 |
| | dATP 20 µM<br>dCTP 150 µM<br>TTP 50 µM | ND | total: ND<br>blue: ND<br>white: 1.8 | white<br>C -> T 12<br>G 1<br>A 21 |
| G<br>(C-reaction) | dATP 100 µm<br>dGTP 100 µM<br>TTP 100 µM | 74 % | total: 2.6<br>blue: 2.4<br>white: 3.8 | G -> T 0<br>G 1<br>A 58 |
| | dATP 150 µM<br>dGTP 100 µM<br>TTP 0.2 µM | 9 % | total: ND<br>blue: ND<br>white: 1.2 | white<br>G -> T 5<br>C 26<br>A 8 |

The equilibrium between the different point mutations is altered by changing the concentrations of the "wrong" nucleotides in the misincorporation reaction as shown in Table 3. Table 3 characterizes the mutagenesis in respect of mutant yields, average number of base substitutions per template, and the ratio of different mutations when the three misincorporating nucleotides are used simultaneously at various

concentrations. The G/T and T/G mismatches are formed very easily, and to avoid multiple mismatches the concentrations of the corresponding misincorporating nucleotides must be very low. A correlation is found between the total yield of mutant clones and the number of base substitutions in an average mutant. The number of mutations per template can be regulated by adjusting the nucleotide concentrations at the misincorporation step as shown in Table 3.

When the frequency of mutants is around 60%, each mutant template contains on the average 1.8 base substitutions. Since about one fourth of all possible base substitutions to the amino acid codons are silent, and thus preserve the original amino acid, the value above corresponds to only slightly more than one amino acid substitution per molecule on the average. This is close to optimal, considering that several amino acid changes will be conservative mutations which do not cause much difference in the properties of the polypeptide.

The position and length of the target region to be mutagenized can be varied in a controlled manner. This is done by regulating the extent of limited elongation, simply by increasing (to mutagenize sequences further away from the primer) or decreasing (to make mutations closer to the primer) the concentration of the limiting nucleotide and then combining these aliquots.

Example 2: Complete mutagenesis of the α-amylase from Bacillus stearothermophilus

The α-amylase gene from B. stearothermophilus ATCC 12980, originally cloned in EMBL3 vector, is subcloned as a 1.9 kb fragment from a partial Sau3A digest, first to the BamHI site of pUC8 and then as a Sma I-HindIII fragment to the phage vectors M13mp18 and M13mp19. Figure 3 shows the constructions obtained, M13mp18α and M13mp19α. The 1.9 kb long insert contains 234 nucleotides from the 5′ noncoding promoter region of the α-amylase gene, the complete coding region and 13 nucleotides of the 3′ noncoding region. Since the sequence of the insert is known, synthetic 20-mer oligonucleotides complementary to the target sequence at about 200 nucleotide intervals are used as primers. Both strands can be mutagenized separately.

Uracil-containing single-stranded M13mp18α and M13mp19α are prepared by the method described in Example 1. The oligonucleotide primers mentioned above are annealed to the uracil-containing template as described in the previous example. The limited elongation, misincorporation and all-the-way-around elongation are performed as described. Either uracil-containing M13mp18 α or M13mp19 α can be used as a template, or they can both be used as templates in separate tubes and together with their own primers. If both strands are mutagenized, the method is even more random and the balance between the different base substitutions is better. A suitable ung+ dut+ strain of E. coli, such as JM 109, is transfected with the mutagenized DNA and the cells are plated on glucose minimal medium/H-top amylopectic azure (0.25%, Calbiochem) plates, with or without IPTG at 480 ug/plate. Expression of functional recombinant α-amylase by E. coli is detected as halos around the plaques.

JM109 is grown overnight at 37°C in 2 × TY, 250 rpm, and diluted 1:100 in 2 × TY. This suspension is pipetted in 200 ul aliquots to the wells of sterile microtiter plates (Sterilan). The cells in each well are infected by a single plaque picked from the plates with sterile toothpicks. The microtiter plates are incubated in a humid chamber at 37°C, with shaking at 250 rpm, for 18 h.

The α-amylase activity (Bernfeld, P., Amylases, α and β . 1, Methods Enzymol., 149-158, 1955) is determined by pipetting to empty microtiter plates (Nunc): 90 ul substrate solution, containing 1.1% Zulkowsky soluble starch in 0.2M sodium acetate buffer of the desired pH, 5 mM $CaCl_2$, 50 mM NaCl and 10 ul of suitably diluted enzyme-containing sample from the overnight incubation on a microtiter well. After incubation at 65°C for 15 min, 150 µl dinitrosalicylate (DNS) is added and the samples are incubated for 10 min at 100°C (vapour of boiling water), cooled to room temperature and the absorbance at 540 nm is measured in a Multiskan MCC/340 spectrophotometer (Labsystems) against a zero-sample with 10 µl of water replacing the enzyme-containing sample. In a control sample the DNS reagent (150 µl) is added to 90 µl of substrate solution and the enzyme sample is added last.

The wild type B. stearothermophilus α-amylase has an optimum pH of 5.5 and a temperature optimum of about 80°C. Screening for mutant enzymes with higher or lower activity than the wild type is done by determining the activities at pH 5.5, 65°C as described above. Screening for mutant enzymes with increased acitivity at pH 3.5 requires that the pH at the activity determination is 3.5 and it is achieved by adjusting the pH of the substrate solution. Mutants with increased thermostability are screened for by including an incubation step at a high temperature (such as 95°C for 1 hour) before the determination of α-amylase activities under optimal conditions.

An alternative method, or a method to be used for preliminary screening for mutant amylases is a

replica plating test. E. coli JM109 transfected with mutagenized M13mp18α or M13mp19α is grown on glucose minimal/H-top plates supplemented with 0.25% amylopectin azure (Calbiochem). After the formation of plaques and production of α-amylase, a dry, sterile Whatman 1 paper is tightly pressed on the plate to trap the enzyme, and then the paper is transferred into good contact with a substrate plate and incubated at 65°C for 2 or more hours. For screening of α-amylases of increased activity at pH 3.5, the substrate plate contains 0.25% soluble starch (Zulkowsky) or insoluble starch (Maizena) on 1% agarose plate in pH 3.5 buffer.

Mutant clones are also characterized by sequencing to identify the mutation. Figure 4 shows the 515 amino acids long protein sequence of B. stearothermophilus α-amylase, and some of the amino acid replacements obtained from the mutagenesis are shown on top of the wild type sequence. These mutants have been identified from partial mutant libraries generated from particular primers.

Several of the mutants obtained have diminished activity, and some of them are inactive. The method can thus also be used for mapping of the functional regions of proteins. For example, the mutants Ile 354 → Leu, Tyr 370 → Ser, Ile 428 → Phe, Ser 435 → Phe, among several others, are inactive towards starch.

One particular mutant, Tyr 265 → (Ser, has an altered pH-behaviour as shown in Figure 5. The activities have been measured as described earlier, at 65°C in sodium acetate buffers of pH 3.5 - 6, and in PIPES buffers (piperazine - N, N'-bis [2-ethane sulfonic acid]) in the pH range 6-8. Compared to the activity of the wild type enzyme (100% activity), the mutant enzyme shows about 52% activity in the pH range 5.0 - 8.0, but at pH 4.5 it has only about 33% and at pH 4.0 about 11% of the activity of the wild type enzyme. This demonstrates that the activity versus pH curve can be altered by a single point mutation.

The nucleic acid template for mutagenesis can also be RNA. In that case a complementary primer is hybridized to the RNA, the limited elongation and misincorporation steps are performed as described but using reverse transcriptase instead of Klenow enzyme in the elongation, and then double stranded cDNA is made using RNase H and DNA polymerase I as described by Teeri, T., Kumar, V., Lehtovaara, P. and Knowles, J., Construction of cDNA libraries by blunt end ligation: high frequency cloning of long cDNAs from filamentous fungi, 164 Anal. Biochem., 60-67 (1987). The double stranded cDNA is ligated to a suitable vector and expressed in a suitable host. Any mutation generated in the first strand of cDNA will be copied to the second strand, so that each of the progeny strands will contain the mutation.

In place of the specific oligonucleotide primers used for limited elongation in this example, it is possible to use only one primer. If the sequence of the target is unknown, the primer can be hybridized to the bordering vector sequences. Limited elongation can be carried out using suitable concentrations of a particular limiting nucleotide to produce families of molecules of defined length, for example 200, 400, 600, 800 etc. nucleotides. The elongation is monitored by electrophoresis of radioactively labeled samples together with size markers on sequencing gels or alkaline agarose gels (as described in Maniatis et al, 1982), and the desired fractions are combined. A second limited elongation step is then carried out as described in Example 1 in four separate reactions, each with a different limiting nucleotide.

Alternatively, the misincorporation step can be carried out in the presence of one nucleotide or thionucleotide only. The Klenow polymerase or any other polymerase can be used instead of reverse transcriptase if thionucleotides are misincorporated. The final elongation step, which gives molecules able to transform the host, can also be modified in any of the following ways:

(1) Any polymerase, for instance the Klenow enzyme, can be used instead of T4 polymerase. If T4 polymerase is not used, the p32 protein is unnecessary.

(2) Additional primers can be hybridized to the vector sequences before the final elongation step to increase the amount of full length molecules.

(3) After the misincorporation, the final elongation step can be replaced by hybridization of the uracil template to its complementary strand, obtained by restriction enzyme cleavage of the vector replicative form. The remaining gaps are filled in with polymerase and sealed with ligase.

The foregoing general discussion and experimental examples are intended to be illustrative of the present invention, and are not to be considered as limiting. Other variations within the spirit and scope of this invention are possible and will present themselves to those skilled in the art.

## Claims

1. A method for introducing random point mutations to any nucleic acid, **characterized** by

(a) preparation of the target nucleic acid to be mutagenized in the form of a single stranded template;

(b) extension of an oligonucleotide hybridized to this template to generate a population of DNA molecules, each copied from the template, and comprising molecules terminating at all possible nucleotide positions within a predetermined region;

(c) misincorporation of nucleotides opposite to each position of the template using polymerase and the variable 3′ ends generated in (b) as primers;

(d) completion of the DNA molecules generated in (c) to forms that can be amplified by molecular cloning in a suitable host;

(e) expression of the mutagenized DNA using a suitable host vector system allowing the screening for an altered gene or gene product.

2. The method of claim 1, **characterized** in that the target nucleic acid codes for a polypeptide.

3. The method of claim 1, **characterized** in that the target nucleic acid contains a region which functions in regulating gene expression.

4. A method for preparation of a novel polypeptide including those not found in nature, **characterized** by

(a) preparation of the target nucleic acid coding for the polypeptide to be mutagenized in the form of a single stranded template;

(b) extension of an oligonucleotide hybridized to the template to generate a population of molecules, each copied from the template, and comprising molecules terminating at all possible nucleotide positions within a predetermined region;

(c) misincorporation of nucleotides opposite to each position of the template using polymerase and the variable 3′ ends generated in (b) as primers;

(d) completion of the molecules generated in (c) to forms that can be amplified by molecular cloning in a suitable host;

(e) expression of the mutagenized nucleic acids, using a suitable host vector system allowing the screening for an altered gene product;

(f) screening for a clone expressing a mutant polypeptide which functions better than the unmutagenized polypeptide under the desired conditions.

5. A method for altering the structure and function of nucleic acid regions which function in regulating gene expression, **characterized** by

(a) preparation of the target nucleic acid in the form of a single stranded template, containing the regulatory region to be mutagenized and a region not to be mutagenized, coding for an easily quantitated gene product whose expression levels correlate to the structure of the regulatory region;

(b) extension of an oligonucleotide hybridized to the template to generate a population of molecules, each copied from the template, and comprising of molecules terminating at all possible nucleotide positions within a predetermined region;

(c) misincorporation of nucleotides opposite to each position of the template using polymerase and the variable 3′ ends generated in (b) as primers;

(d) completion of the molecules generated in (c) to forms that can be amplified by molecular cloning in a suitable host;

(e) expression of the mutagenized nucleic acids, using a suitable host vector system allowing the screening for a clone where the mutagenized gene regulatory region functions better than the unmutagenized regulatory region under the desired conditions.

FIG. 1a

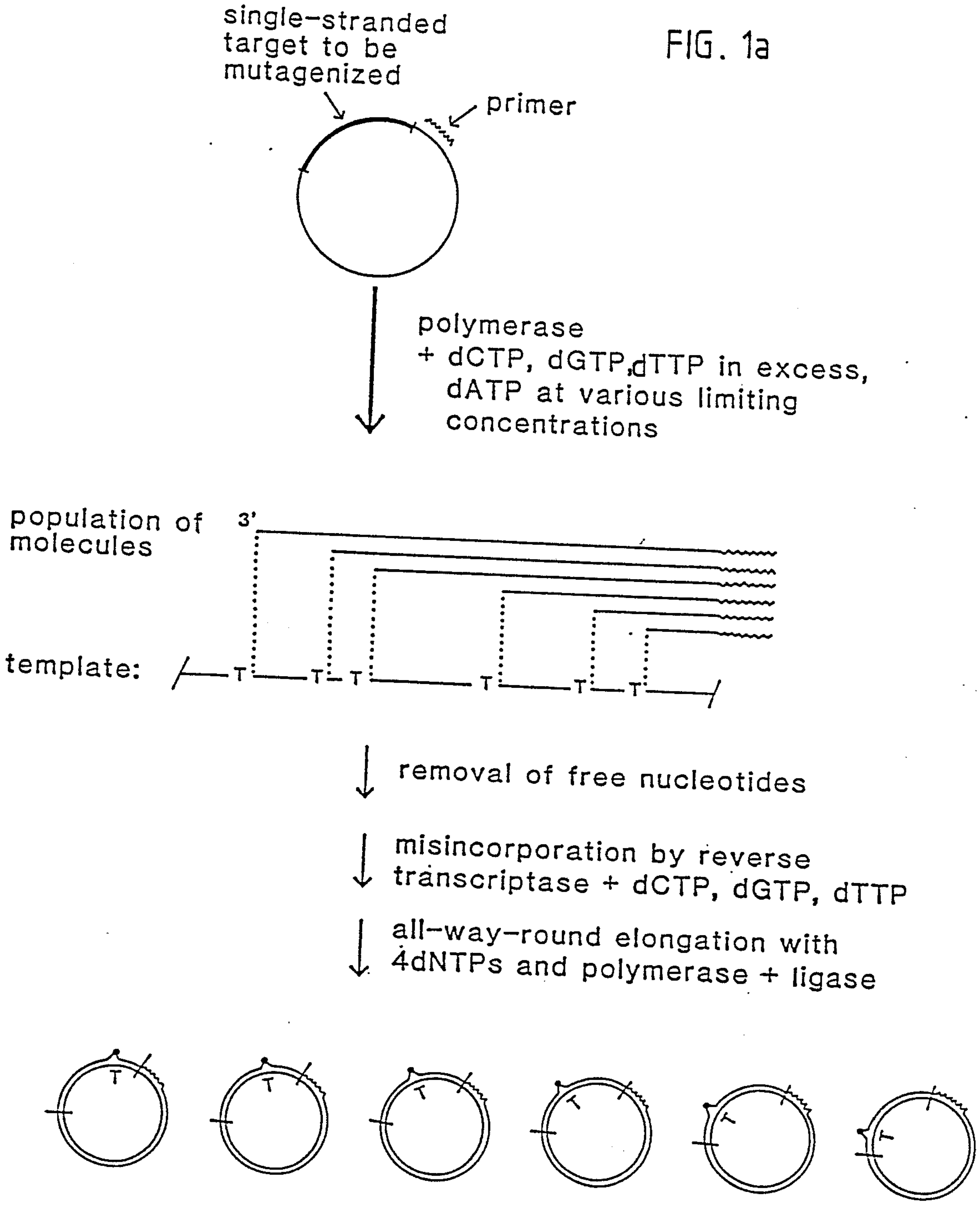


population of mutant molecules with T/C, T/G or T/T mismatches

FIG. 1b

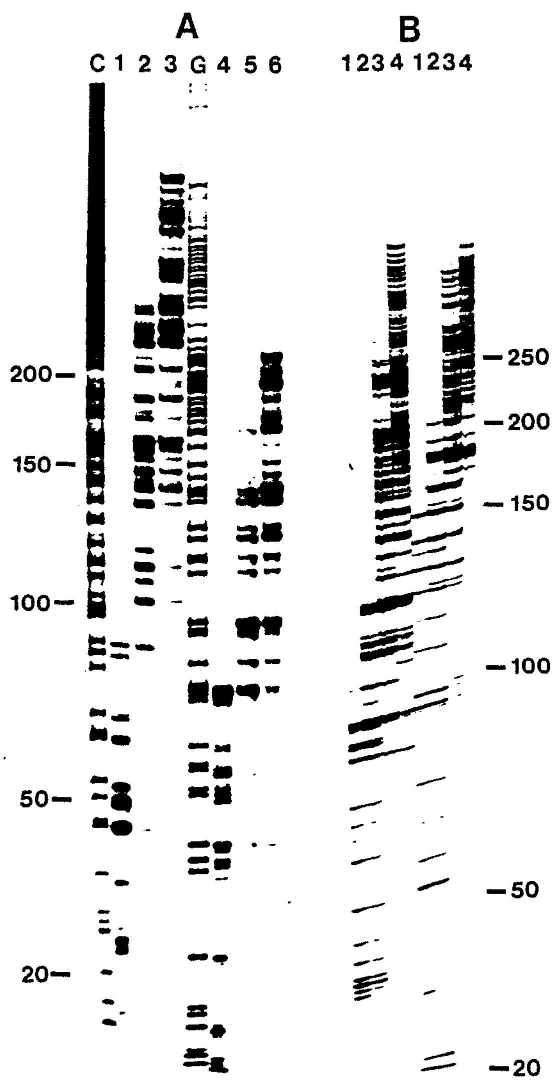

```
                                             G           G
A    A       A        A         CCA   C A    C      C     C  CG  A
GCT ATG ACC ATG ATT ACG(CCA AGC TTG CAT GCC TGC AGG TCG ACT CTA GAG
    Met Thr Met Ile Thr(Pro Ser Leu His Ala Cys Arg Ser Thr Leu Glu

                                  T  CC              CT       G  C
 XC          CT         C     C C G  AA     CA  A    AC TGG AAC XAC
GAT CCC CGG GTA CCG AGC)TCG AAT TCA CTG GCC GTC GTT TTA CAA CGT CGT
Asp Pro Arg Val Pro Ser)Ser Asn Ser Leu Ala Val Val Leu Gln Arg Arg

     G                           T                   T   T   TG
 T   C  T                C  T    G   CC TTC  C  TGG C G CTG TGC  X
AGT AAC A    TT   C AAG AGC G   TC   AA GGA TAT GCC ATC AAC ACA  T
GAC TGG GAA AAC CCT GGC GTT ACC CAA CTT AAT CGC CTT GCA GCA CAT CCC
Asp Trp Glu Asn Pro Gly Val Thr Gln Leu Asn Arg Leu Ala Ala His Pro

    GG       T   TT  G
 G  CCT C T GCT GCC T C     G T T    TT CT   C  T
 C  AAA A A CAA CAA AAA T C CAA A G AGA AA   A  C
CCT TTC GCC AGC TGG CGT AAT AGC GAA GAG GCC CGC ACC GAT CGC CCT TCC
Pro Phe Ala Ser Trp Arg Asn Ser Glu Glu Ala Arg Thr Asp Arg Pro Ser

                                                    primer
```

FIG. 2

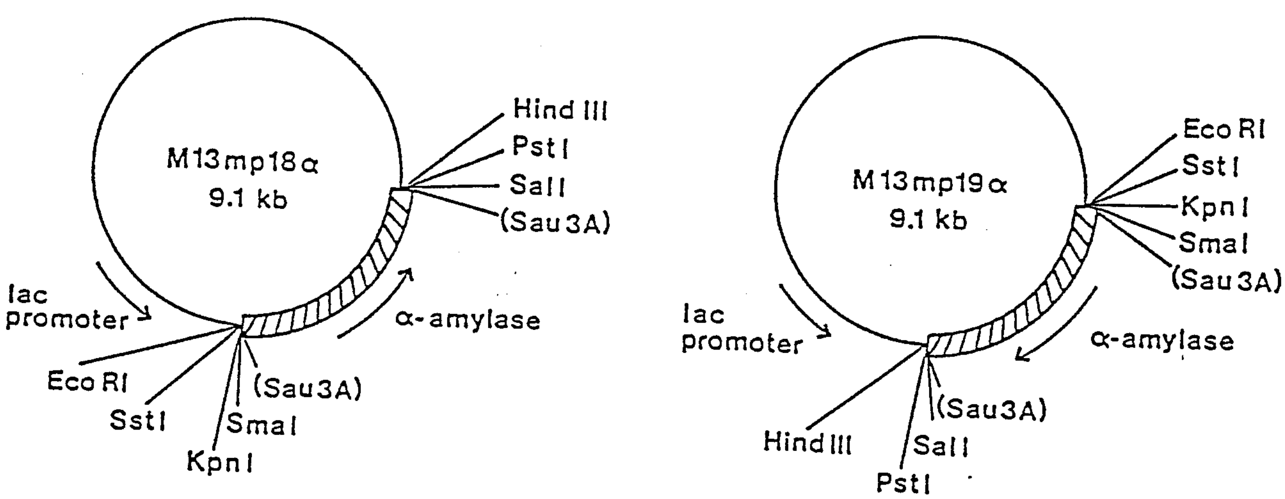


FIG. 3

```
              S  AA P                                 A  S     C
            V F  VV SF PN     YIFC             T   R RV  C  FV F   D
AAPFNGTMMQYFEWYLPDDGTLWTKVANEANNLSSLGITALWLPPAYKGT SRSDVGYGVYDLYDLGEFNQKGAVRTKYGTKAQYLQAIQAAHAAGMQVYA
1                                                50                                               100

                         HT G    S  PT
DVVFDHKGGADGTEWYDAVEVNPSDRNQEISGTYQIQAWTKFDFPGRGNT YSSFKWRWYHFDGVDWDESRKLSRIYKFRGIGKAWDWEVDTENGNYDYLM
101                                              150                                              200

                                                         M
                                                         T   S  DF
                                                 F    PP Q   A  VS           F             F
YADLDMDHPEVVTELKSWGKWYVNTTNIDGFRLDAVKHIKFSFFPDWLSY VRSQTGKPLFTVGEYWSYDINKLHNYIMKTNGTMSLFDAPLHNKFYTASK
201                                              250                                              300

                                   P
                                   H
                              APA CLT     A   N    S  L              SS
SGGTFDMRTLMTNTLMKDQPTLAVTFVDNHDTEPGQALOSWVDPWFKPLA YAFILTRQEGYPCVFYGDYYGIPQYNIPSLKSKIDPLLIARRDYAYGTQH
301                                              350                                              400

                                         N
             V    N        F      F   F  QH                                              P
DYLDHSDIIGWTREGVTEKPGSGLAALITDGPGGSKWMYVGKQHAGKVFY DLTGNRSDTVTINSDGWGEFKVNGGSVSVWVPRKTTVSTIAWSITTRPWT
401                                              450                                              500

DEFVRWTEPRLVAWP
501          515
```

FIG. 4

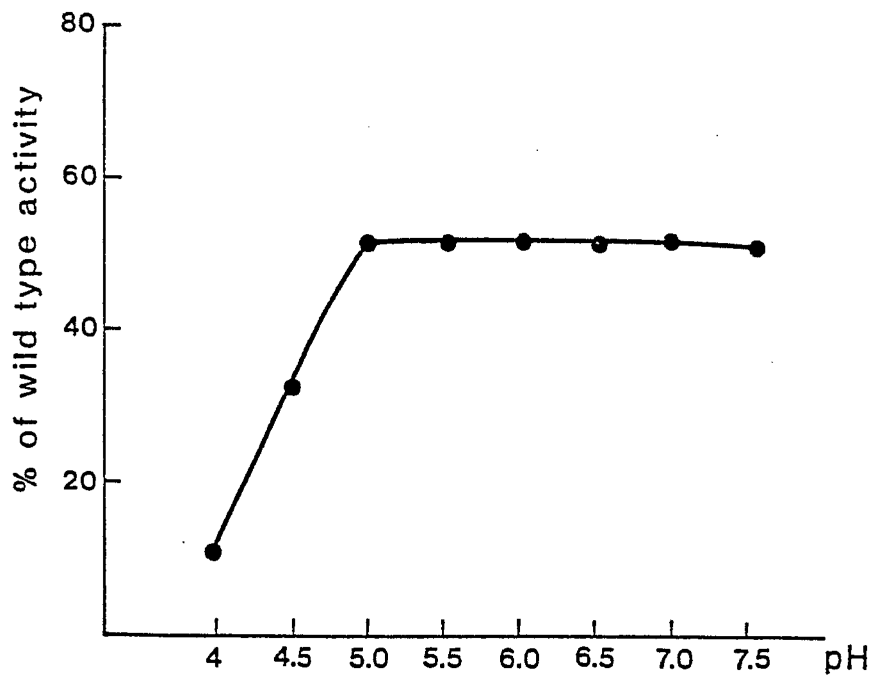


FIG.5

**Andrejewski, Honke & Partner** **Patentanwälte**

European Patent Attorneys
Diplom-Physiker
Dr. Walter Andrejewski
Diplom-Ingenieur
Dr.-Ing. Manfred Honke
Diplom-Physiker
Dr. Karl Gerhard Masch

4300 Essen 1, Theaterplatz 3, Postf. 100254

European Patent Office

8000 München 2

EPA EPO-OEB
DG 1
01-06-1988

Ihr Zeichen

Unser Zeichen
68 194/Fe+

May 26, 1988
Fr.: 25.6; 25.7.88

Betrifft: Patent Application No. 88 105 163.5
Suomen Sokeri Oy

With reference to the communication of May 20, 1988 it is requested to correct the following mistakes in the documents originally filed:

On page 9, line 12, a parenthesis should be added before "Lys 54".

On page 9, line 15, the page numbers should be "739-773".

On page 17, line 24, the parenthesis "(T)" should be "(T-)" and the amount of carrier "12 ug" should be "2 µg".

On page 21, in column T the amount of TTP should be "100 µM" and in column G, the amount of dATP should be "100 µM".

On page 28, line 8, "DNA molecules" should be amended to "molecules".

Representative